(19) Europäisches Patentamt
European Patent Office
Office européen des brevets

(11) EP 0 418 171 B1

(12) **FASCICULE DE BREVET EUROPEEN**

(45) Date de publication et mention de la délivrance du brevet:
**30.10.1996 Bulletin 1996/44**

(51) Int Cl.[6]: **G01F 25/00**

(21) Numéro de dépôt: **90420369.2**

(22) Date de dépôt: **07.08.1990**

(54) **Procédé d'étalonnage d'un débimètre à réponse impulsionnelle**

Kalibrierungsverfahren für einen Impulsantwort-Durchflussmesser

Calibration method for an impulse response flowmeter

(84) Etats contractants désignés:
**AT BE CH DE DK ES FR GB GR IT LI LU NL SE**

(30) Priorité: **16.08.1989 FR 8911083**

(43) Date de publication de la demande:
**20.03.1991 Bulletin 1991/12**

(73) Titulaire: **HOSPAL INDUSTRIE**
**F-69883 Meyzieu Cédex (FR)**

(72) Inventeur: **Fond, André**
**F-69330 Meyzieu (FR)**

(56) Documents cités:
**EP-A- 0 298 587**
**DE-A- 3 336 359**
**FR-A- 2 594 221**
**US-A- 4 831 866**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 418 171 B1

**Description**

La présente invention concerne la mesure du débit d'un fluide circulant dans un circuit au moyen d'un débitmètre à réponse impulsionnelle et, plus précisément, l'étalonnage d'un tel débitmètre.

L'invention trouve une application particulièrement intéressante dans la mesure du débit du liquide de dialyse circulant dans le circuit de dialyse d'un rein artificiel.

De façon classique, un rein artificiel comprend un hémodialyseur séparé par une membrane semi-perméable en deux compartiments, l'un de ces compartiments étant relié à un circuit sang prévu pour être raccordé au circuit vasculaire d'un patient souffrant d'insuffisance rénale, l'autre compartiment étant relié à un circuit de liquide de dialyse prévu pour être parcouru par un liquide de dialyse. Au cours d'une séance de dialyse, les impuretés du sang, que le patient ne peut éliminer naturellement, migrent par diffusion au travers de la membrane et passent dans le liquide de dialyse circulant dans le circuit de dialyse. En outre, la séance de dialyse est généralement mise à profit pour retirer du patient une certaine quantité d'eau plasmatique par convection au travers de la membrane, cette convection étant provoquée par la création d'une différence de pression positive entre les compartiments sang et liquide de dialyse de l'hémodialyseur. La quantité d'eau retirée au patient, ainsi que le débit auquel cette eau est retirée doivent pouvoir être controlés précisément.

Pour effectuer ce contrôle, il est connu de disposer sur le circuit du liquide de dialyse d'un rein artificiel un débitmètre en amont et un débitmètre en aval de l'hémodialyseur, les indications fournies par ces débitmètres étant utilisées classiquement de deux façons : soit pour fournir, par simple différence, une mesure directe du débit de l'eau plasmatique retirée au patient et pour asservir une pompe disposée sur le circuit du liquide de dialyse de façon que ce débit soit égal a une valeur déterminée ; soit pour asservir une pompe disposée sur le circuit du liquide de dialyse de façon que les débits à l'entrée et à la sortie de l'hémodialyseur soient identiques, une autre pompe permettant d'extraire du circuit de liquide de dialyse, entre les deux débitmètres, une quantité de liquide correspondant à la quantité d'eau plasmatique qu'on souhaite retirer du patient.

Quel que soit le mode de contrôle utilisé, il va de soit que sa précision dépend de l'étalonnage des débitmètres.

Le brevet US 4 889 635, qui décrit le second des modes de contrôle évoqué plus haut, propose un procédé d'étalonnage consistant à relier directement en série, au moyen d'une canalisation de dérivation, deux débitmètres à réponse impulsionnelle disposés respectivement en amont et en aval de l'hémodialyseur lors du fonctionnement normal de l'hémodialyseur ; à compter pendant une phase d'étalonnage de durée déterminée, le nombre des impulsions émises par chacun des débitmètres ; et, à calculer, à partir de ce nombre d'impulsions, un coefficient d'étalonnage.

Ce procédé présente l'inconvénient que sa précision dépend directement de la longueur de la phase d'étalonnage : plus cette phase est longue, moins l'influence d'éléments perturbateurs (bulles, etc...) et moins l'imprécision liée au décalage entre le début (respectivement, la fin) de la phase d'étalonnage et la première (respectivement, la dernière) impulsion comptée, sont sensibles.

Or une phase d'étalonnage longue, si elle n'est pas un handicap insurmontable en début de séance, est en revanche rédhibitoire si, dans le but d'obtenir une grande précision dans la mesure du débit des liquides, elle doit être répétée à plusieurs reprises au cours d'une séance de dialyse, cette opération étant rendue nécessaire avec les débitmètres connus actuellement qui dérivent sensiblement au cours d'une telle séance (qui dure généralement environ quatre heures) : en effet, l'efficacité thérapeutique de la dialyse s'oppose à ce que la circulation de liquide de dialyse dans l'hémodialyseur soit interrompue trop longtemps au cours de la séance.

La présente invention vise donc à proposer un procédé d'étalonnage pour un débitmètre délivrant un signal électrique à impulsions, permettant d'obtenir une précision de mesure élevée, indépendante du temps d'étalonnage.

L'invention vise également à offrir un procédé permettant l'étalonnage d'un débitmètre pendant un temps très court, tout en neutralisant l'influence d'éléments perturbateurs accidentels.

L'invention vise aussi à proposer un procédé particulièrement adapté à l'étalonnage de débitmètres rotatifs à réponse impulsionnelle.

L'invention vise à offrir, en outre, un procédé propre à assurer l'étalonnage de débitmètres équipant, plus particulièrement, un circuit de liquide de dialyse d'un rein artificiel.

Pour atteindre les buts énoncés ci-dessus, la présente invention propose un procédé d'étalonnage d'un premier débitmètre au moyen d'un second débitmètre, consistant à relier en série le premier débitmètre au second débitmètre, chaque débitmètre délivrant un signal électrique à impulsions (S) dont la fréquence correspond à une valeur donnée de débit d'un fluide circulant dans un circuit, caractérisé en ce qu'il comporte les étapes de :

- mesurer pour chaque débitmètre, pendant une phase d'acquisition de données d'une durée moyenne (Ta), chaque temps élémentaire ($T_7$(i); $T_8$(i)) séparant deux impulsions décalées d'un pas de mesure (P) déterminé,
- compter, pour chaque débitmètre, le nombre d'impulsions (n ; m) émises pendant la phase d'acquisition (Ta) ;
- calculer, à partir des temps élémentaires mesurés et du nombre d'impulsions comptées, la période ($Tm_7$ ; $Tm_8$) moyenne d'étalonnage de chaque

débitmètre ; et

- déterminer un coefficient d'étalonnage à partir d'une comparaison entre la période ($Tm_7$) moyenne d'étalonnage du premier débitmètre et la période ($Tm_8$) moyenne d'étalonnage du second débitmètre prise comme valeur de référence.

Grâce à ce procédé, on élimine l'imprécision liée, dans le procédé d'étalonnage mentionné plus haut, à la prise en compte, dans la durée de l'étalonnage proprement dite, du temps correspondant au décalage entre le début (respectivement, la fin) de la phase d'acquisition de données et la première (respectivement, la dernière) impulsion comptée. En outre, ce procédé permet un traitement ultérieur des données recueillies, permettant en particulier de minimaliser l'importance des données erratiques ou de les éliminer.

Dans un mode préféré de réalisation, pour lequel chaque débitmètre est du type rotatif à N impulsions engendrées par tour de rotor, le procédé d'étalonnage consiste, pour déterminer la période moyenne d'étalonnage :

- à calculer des périodes de rotation successives ($ST_7(i)$ ; $ST_8(i)$) en procédant, pour chacune d'entre-elles, à la sommation de N temps élémentaires ($T_7(i)$ ; $T_8(i)$) consécutifs,
- et à traiter les périodes de rotation ($ST_7(i)$ ; $ST_8(i)$) pour déterminer la période moyenne d'étalonnage.

Grâce à ce mode de réalisation, on peut, d'une part, s'affranchir de la dispersion éventuelle des valeurs des données acquises, due aux caractéristiques mécaniques ou géométriques du débitmètre (cette dispersion pouvant être gênante lorsqu'on envisage de soumettre ces données à un traitement statistique ultérieur), et, d'autre part, disposer dans un temps très court d'un nombre d'informations suffisant pour effectuer un étalonnage très précis.

L'invention a également pour objet un dispositif de contrôle et de traitement pour la mise en oeuvre du procédé d'étalonnage mentionné ci-dessus, pour des débitmètres équipant un circuit de dialyse d'un rein artificiel, caractérisé en ce qu'il comporte :

- un microprocesseur connecté par une liaison à deux débitmètres,
- un compteur pour mesurer chaque temps élémentaire ($T_7(i)$ ; $T_8(i)$) séparant deux impulsions émises par chacun des débitmètres,
- un moyen de comptage des impulsions émises pendant les temps élémentaires ($T_7(i)$; $T_8(i)$) considérés,

  - et des moyens de calcul pour calculer, à partir des temps élémentaires ($T_7(i)$; $T_8(i)$) mesurés, une période moyenne d'étalonnage ($Tm_7$ ; $Tm_8$) pour chaque débitmètre, et pour calculer, à partir d'une comparaison des périodes moyennes d'étalonnage ($Tm_7$ ; $Tm_8$) calculées, un coefficient d'étalonnage.

D'autres caractéristiques et avantages de l'invention ressortent de la description faite ci-dessous en référence aux dessins annexés, sur lesquels :

La figure 1 est un schéma d'un rein artificiel équipé de débitmètres à réponse impulsionnelle auxquels le procédé d'étalonnage selon l'invention est appliquable ;
La figure 2 présente des chronogrammes explicitant le procédé d'étalonnage selon l'invention ;
La figure 3 présente un chronogramme illustrant un détail caractéristique du procédé de l'invention ;
La figure 4 présente un diagramme montrant la dispersion des mesures réalisées selon le procédé de l'invention, pour un débitmètre rotatif ;
Les figures 5A et 5B présentent des histogrammes illustrant un autre détail caractéristique du procédé selon l'invention ; et
La figure 6 illustre un algorithme simplifié décrivant le procédé d'étalonnage selon l'invention.

Le rein artificiel représenté sur la figure 1 comporte un circuit 1 de liquide de dialyse relié au premier compartiment 2 d'un hémodialyseur 3. De façon classique, l'hémodialyseur 3 comporte un second compartiment 4, séparé du premier par une membrane semi-perméable 5 et relié au patient par l'intermédiaire d'un circuit extracorporel de sang 6.

Le circuit 1 comporte une entrée A reliée à une source de liquide de dialyse (non représentée), ainsi qu'une sortie F connectée à des moyens d'évacuation ou de recyclage non représentés. Le circuit de dialyse 1 est équipé, respectivement, en amont et en aval de l'hémodialyseur 3, de débitmètres 7, 8 mesurant la quantité du liquide qui circule en émettant chacun une impulsion électrique au passage d'une fraction déterminée de liquide de dialyse. De préférence, mais non exclusivement, les débitmètres 7, 8 sont de nature identique et du type rotatif. Chaque débitmètre délivre un nombre N d'impulsions électriques par tour de rotor. Chaque impulsion est émise au passage, devant un détecteur fixe, d'une pale ou d'un repère porté le rotor. Un tel signal électrique est transmis par une liaison 9 à un dispositif 11 de contrôle et de traitement, dont la fonction apparaîtra plus clairement dans la suite de la description.

Le circuit 1 comporte des pompes de circulation 12, 13 placées, par exemple, respectivement entre le débitmètre amont 7 et l'entrée C de l'hémodialyseur et entre la sortie D de l'hémodialyseur et le débitmètre aval 8. Le circuit 1 comporte également une conduite de dérivation 14 comprenant une vanne 16 et reliée, d'une part, en un point B du circuit, à l'entrée C de l'hémodialyseur, par l'intermédiaire d'une vanne 15 et, d'autre part, en un point E du circuit, à la sortie D de l'hémodialyseur, par

l'intermédiaire de la pompe de circulation 13.

Pour procéder à l'étalonnage de chacun des deux débitmètres, l'hémodialyseur 3 est court-circuité par la fermeture de la vanne 15 et l'ouverture de la vanne 16, tandis que la pompe de circulation 13 est en position d'arrêt. Le liquide de dialyse est donc mis en circulation par la pompe 12 dans le circuit formé par les tronçons A, B, E, F. Pendant cette phase d'étalonnage, le débit du liquide circulant dans le débitmètre amont 7 est rigoureusement égal au débit du liquide circulant dans le débitmètre aval 8, dans la mesure où le circuit ainsi formé ne possède aucun point de perte ou de gain de liquide de dialyse.

Tel que cela ressort plus précisément de la figure 2, le procédé d'étalonnage selon l'invention consiste, pendant une phase d'acquisition de données d'une durée déterminée Ta, à mesurer, pour chaque débitmètre 7, 8, chaque temps élémentaire $T_7(i)$ et $T_8(i)$ séparant deux impulsions S décalées d'un pas de mesure P déterminé. Chaque impulsion est définie comme étant la partie du signal séparant, par exemple, deux fronts montants successifs. Dans l'exemple illustré sur la figure 2, le pas de mesure P est égal à i, de sorte que chaque temps élémentaire est égal à la durée d'une impulsion. Dans l'exemple illustré sur la figure 3, le pas de mesure P est 4, de sorte que chaque temps élémentaire est égal à la durée de quatre impulsions.

On note que le temps Ta est un temps moyen d'acquisition de données. En effet, une telle phase d'acquisition est destinée à permettre l'enregistrement d'un nombre entier et maximal de temps élémentaires, dont la somme ne correspond généralement pas à la durée Ta, comme cela ressort clairement de la figure 2.

Le procédé consiste, également, pour chaque débitmètre 7, 8, à compter le nombre d'impulsions émises, respectivement n et m, pendant le temps d'acquisition Ta et à calculer, ensuite, les périodes moyennes d'étalonnage Tm.

Selon une première variante de réalisation, chaque période moyenne d'étalonnage $Tm_7$, $Tm_8$ est calculée en effectuant la somme des temps élémentaires sur le nombre d'impulsions compté, soit :

$$Tm_7 = \frac{1}{n} \cdot \sum_{i=1}^{n} T_7(i)$$

et :

$$Tm_8 = \frac{1}{m} \cdot \sum_{i=1}^{m} T_8(i)$$

Selon une seconde variante préférée de réalisation, dans laquelle chaque débitmètre est du type rotatif à N impulsions engendrées par tour de rotor, les périodes moyennes d'étalonnage sont déterminées en tenant compte de dispersions dues aux caractéristiques mécaniques et/ou géométriques de ces débitmètres. En effet, selon cette variante, chaque temps élémentaire mesuré entre deux impulsions successives correspond à l'intervalle de temps séparant le passage, devant le détecteur, de deux repères ou deux pales successives. Aussi, les temps élémentaires ne sont pas identiques, dans la mesure ou les N pales ou repères ne se trouvent pas régulièrement répartis angulairement sur le rotor. Un tel phénomène apparaît clairement sur la figure 4 qui montre un diagramme représentant, à titre d'exemple, les différences de valeurs entre les temps élémentaires $T_7$ en fonction du rang considéré des pales d'un débitmètre. Dans l'exemple illustré, le débitmètre comporte quatre pales $P_1$ à $P_4$.

Le procédé d'étalonnage selon l'invention vise à s'affranchir d'un tel décalage d'implantation angulaire pour permettre de déterminer une période d'étalonnage la plus précise possible. A cet effet, le procédé selon l'invention vise, pour chaque débitmètre, à calculer une série de périodes de rotation successives $ST_7(i)$ et $ST_8$ (i). Tel que cela apparaît plus précisément sur la figure 2, ces périodes de rotation sont déterminées en procédant, pour chacune d'entre elle, à la somme de N temps élémentaires consécutifs et au décalage d'un temps élémentaire à chaque période de rotation successive, de manière à éliminer de la somme le premier temps élémentaire de la période précédente, et à ajouter à la somme le temps élémentaire subséquent au dernier temps élémentaire pris en compte pour la période précédente. En d'autre termes, deux périodes de rotation consécutives $ST_7(i)$, $ST_7(i+1)$ (respectivement $ST_8(i)$, $ST_8(i+1)$ se chevauchent sur (N-1) temps élémentaires. On peut aussi prévoir de faire se chavaucher deux périodes de rotation consécutives sur un nombre inférieur de temps élémentaires.

A titre d'exemple, si le nombre N de pales est égal à quatre, chaque période de rotation $ST_7(i)$ ou $ST_8(i)$ est égale à la somme de quatre temps élémentaires. Ainsi, les premières périodes de rotation $ST_7(1)$ et $ST_8(1)$ sont telles que :

$$ST_7(1) = T_7(1) + T_7(2) + T_7(3) + T_7(4)$$

et :

$$ST_8(1) = T_8(1) + T_8(2) + T_8(3) + T_8(4)$$

Les secondes périodes de rotation sont calculées en éliminant de la somme le premier temps élémentaire et en prenant en compte le cinquième temps élémentaire, de sorte que :

$$ST_7(2) = T_7(2) + T_7(3) + T_7(4) + T_7(5)$$

et :

$$ST_8(2) = T_8(2) + T_8(3) + T_8(4) + T_8(5)$$

Les périodes de rotation suivantes sont cbtenues en réalisant un décalage identique, jusqu'à la dernière période élémentaire mesurée $ST_7(j)$ et $ST_8(j)$. Le nombre j, k de période de rotation, respectivement des débitmètres 7, 8, est tel que :

$$j = n - (N - 1)$$

$$k = m - (N - 1)$$

Une telle méthode permet donc d'éliminer les effets de la dissymétrie d'implantation angulaire des pales ou des repères sur le rotor, en prenant en compte des périodes de rotation correspondant à un tour de rotation du rotor du débitmètre.

Une autre méthode, pour éliminer les effets d'une telle dissymétrie d'implantation, peut être envisagée, dans le cas où la fréquence du signal impulsionnel est élevée. Selon la figure 3, les périodes de rotation sont définies pour correspondre au temps mis par une même pale ou par un seul et même repère pour accomplir un tour de rotation. Pour N = 4, chaque période de rotation est égale à la durée de 4 impulsions successives. Les figure 5A et 5B montrent respectivement un exemple de distribution des périodes de rotation $ST_7(i)$ et $ST_8(i)$ déterminées pour une phase d'étalonnage donnée Les périodes de rotation sont ensuite traitées de manière déterminer les périodes moyennes d'étalonnage correspondantes. Une première méthode de traitement possible des valeur consiste à effectuer le rapport de la somme des périodes d rotation sur le nombre de périodes pour déterminer la périod moyenne d'étalonnage $Tm_7$, $Tm_8$, soit :

$$Tm_7 = \frac{1}{j} \cdot \sum_{i=1}^{j} ST_7(i)$$

et :

$$Tm_8 = \frac{1}{k} \cdot \sum_{i=1}^{k} ST_8(i)$$

Une telle méthode de traitement apparaît particulièrement adaptée si les périodes de rotation se trouvent distribuées selon une courbe de Gauss, tel que cela est illustré à la figure 5A.

Une autre méthode de détermination de la période moyenne d'étalonnage s'avère plus particulièrement adaptée pour éliminer les erreurs de mesure qui apparaissent clairement sur le diagramme de la figure 5B. Dans cet exemple, on constate que quelques périodes de rotation présentent des valeurs élevées correspondant, notamment, à des artefacts de mesure. Pour éliminer de telles erreurs de mesure, la période moyenne d'étalonnage Tm est déterminée de manière que le maximum de valeurs de périodes de rotation se situe dans un intervalle I centré autour de cette période moyenne Tm. De plus, il peut être prévu que la période moyenne d'étalonnage Tm ainsi déterminée sera validée dans la mesure où le nombre de valeurs de périodes de rotation mesurées, qui sont contenues dans cet intervalle I, est supérieur ou égal à un pourcentage prédéterminé du nombre total de valeurs de périodes de rotation mesurées.

A titre d'exemple, l'étalonnage sera validé si 80% des valeurs de périodes de rotation sont contenues dans l'intervalle I prédéterminé et centré autour de la période moyenne Tm.

De préférence, l'intervalle I est l'intervalle compris entre la valeur de la période moyenne Tm plus le double d'un écart type standard (ECT) et la valeur de la période moyenne Tm moins le double d'un écart type standard (ECT). Un tel écart type standard est déterminé dans des conditions de mesure statique, à partir de l'analyse des informations fournies par le même type de débitmètre utilisé au cours des phases d'étalonnage et de dialyse.

Le procédé d'étalonnage décrit ci-dessus, qui repose sur la mesure des temps élémentaires entre deux impulsions, permet d'obtenir une précision de mesure élevée et indépendante du temps d'étalonnage. Par ailleurs, ce procédé autorise l'obtention d'un grand nombre de données permettant un traitement adapté des mesures pour éliminer les erreurs accidentelles de mesure. Il s'ensuit que le temps d'étalonnage peut être limité à une durée minimale nécessaire à l'acquisition de données suffisantes pour un traitement ultérieur, sans toutefois affecter la précision de mesure, puisque les erreurs susceptibles d'apparaître pendant ce laps de temps très court, sont éliminées par le procédé d'étalonnage selon l'invention. A titre d'exemple, l'expérience

pratique montre que la phase d'étalonnage de deux débitmètres peut être réalisée pendant une durée de l'ordre de dix secondes.

Le procédé d'étalonnage selon l'invention peut être avantageusement mis en oeuvre par des moyens de programmation implantés dans un microprocesseur faisant partie intégrante du dispositif 11 de contrôle et de traitement. Les moyens de programmation comportent, en outre, un algorithme principal d'étalonnage, comme illustré à la figure 6. Cet algorithme présente une phase d'initialisation 100, au cours de laquelle est effectuée successivement l'acquisition des périodes élémentaires de chacun des débitmètres selon, par exemple, la méthode suivante. Un signal d'interruption, qui est transmis au microprocesseur, est engendré à chaque front descendant d'une impulsion. Le signal d'interruption autorise la lecture d'un étage tampon 11a dans lequel est enregistrée en continu la valeur d'un compteur 11b. Au moment de chaque interruption, la valeur du compteur, contenue dans l'étage tampon, est mémorisée, puis retranchée de celle enregistrée précédemment, de manière à déterminer une période élémentaire. Bien entendu, le nombre d'interruptions est enregistré pour connaître le nombre d'impulsions émises pendant les périodes élémentaires.

L'acquisition des périodes élémentaires s'effectue tant que le temps d'enregistrement Ta d'un débitmètre n'est pas écoulé (étape 101).

Lorsque les temps élémentaires de chacun des débitmètres ont été enregistrés successivement, les signaux d'interruption sont inhibés (étape 102), de manière à permettre le traitement des données prises en compte selon le procédé décrit ci-dessus (étape 103).

Si la période moyenne d'étalonnage calculée ne permet pas d'obtenir un pourcentage déterminé de valeurs de périodes de rotation comprises dans l'intervalle choisi centré autour de cette moyenne, un signal d'erreur d'étalonnage est émis et une nouvelle phase d'acquisition de données doit être effectuée (étape 104).

Lorsque les périodes d'étalonnage de chaque débitmètre sont déterminées de manière précise, le dispositif de contrôle 11 calcule un coefficient d'étalonnage qui peut être, par exemple, le rapport entre les deux périodes moyennes d'étalonnage calculées (étape 105). Ce coefficient d'étalonnage permettra de corriger les mesures de débit effectuées par les débitmètres lors de la séance de dialyse.

Tel que cela ressort de la figure 1, pour mettre en oeuvre la séance de dialyse qui suit la phase d'étalonnage initiale, la vanne 16 est fermée tandis que la vanne 15 est ouverte, de sorte que le liquide de dialyse circule dans le circuit A, C, D, F et traverse le compartiment 2 de l'hémodialyseur 3. Selon une forme préférée de fonctionnement, une pompe d'extraction 17 est montée sur le circuit de dialyse, par exemple entre la pompe de circulation 13 et la sortie D de l'hémodialyseur, de manière à extraire du circuit 1 une quantité de liquide de dialyse égale à la quantité d'eau plasmatique à retirer du sang, la pompe 13 étant par ailleurs asservie à la comparaison des indications fournies par les débitmètres 7, 8 pour que les débits des liquides y circulant respectivement soient identiques.

Avantageusement, le débit de liquide de dialyse, lors de la phase d'étalonnage, est choisi pour être sensiblement identique au débit de liquide de dialyse circulant au cours de la séance de dialyse.

De préférence, un coefficient d'étalonnage vrai peut être déterminé, en faisant la demi-somme de deux coefficients calculés dans deux phases successives d'étalonnage. Ce coefficient vrai d'étalonnage correspond à celui qui aurait dû être utilisé pendant la séance de dialyse entre les deux phases d'étalonnage consécutives considérées. Aussi, le dispositif de contrôle 11 détermine un coefficient de correction correspondant à l'écart entre le coefficient vrai et le premier des deux coefficients considéré, permettant de corriger, par ce coefficient de correction, le volume relatif de liquide mesuré par les débitmètres entre les deux phases successives d'étalonnage considérées.

Si la valeur du débit de liquide de dialyse est modifiée de manière significative au cours de la séance de dialyse, une nouvelle phase d'étalonnage doit être réalisée avec cette nouvelle valeur de débit du liquide de dialyse, de manière à déterminer un nouveau coefficient d'étalonnage. Cette phase d'étalonnage peut être effectuée selon le procédé de l'invention puisque la durée d'étalonnage est limitée dans le temps.

Il apparaît donc que le procédé d'étalonnage d'un débitmètre selon l'invention trouve une application particulièrement avantageuse pour des débitmètres équipant un circuit de liquide de dialyse. En effet, le procédé conforme à l'invention permet d'effectuer l'étalonnage de débitmètres pendant un temps très court, tout en éliminant les erreurs accidentelles de mesure, de sorte que l'efficacité de la dialyse reste entière.

L'invention n'est pas limitée aux exemples décrits et représentés et elle est susceptible de modifications et de variantes qui apparaîtront à l'homme de l'art.

**Revendications**

1. Procédé d'étalonnage d'un premier débitmètre (7) au moyen d'un second débitmètre (8), consistant à relier en série le premier débitmètre (7) au second débitmètre (8), chaque débitmètre délivrant un signal électrique à impulsions (S) dont la fréquence correspond à une valeur donnée de débit d'un fluide circulant dans un circuit,
   caractérisé en ce qu'il comporte les étapes de :

   - mesurer pour chaque débitmètre (7, 8), pendant une phase d'acquisition de données d'une durée moyenne (Ta), chaque temps élémentaire ($T_7(i)$; $T_8(i)$) séparant deux impulsions décalées d'un pas de mesure (P) déterminé,

- compter, pour chaque débitmètre (7, 8), le nombre d'impulsions (n ; m) émises pendant la phase d'acquisition (Ta) ;
- calculer, à partir des temps élémentaires mesurés et du nombre d'impulsions comptées, la période ($Tm_7$; $Tm_8$) moyenne d'étalonnage de chaque débitmètre (7, 8) ; et
- déterminer un coefficient d'étalonnage à partir d'une comparaison entre la période ($Tm_7$) moyenne d'étalonnage du premier débitmètre (7) et la période ($Tm_8$) moyenne d'étalonnage du second débitmètre (8) prise comme valeur de référence.

**2.** Procédé d'étalonnage selon la revendication 1, caractérisé en ce qu'il consiste à calculer la période moyenne d'étalonnage ($Tm_7$ ; $Tm_8$) en effectuant le rapport de la somme des temps élémentaires ($T_7$ (i); $T_8$(i)) sur le nombre d'impulsions comptées (n ; m).

**3.** Procédé d'étalonnage selon la revendication 1, adapté à un débitmètre rotatif à N impulsions engendrées par tour de rotor, caractérisé en ce qu'il consiste, pour déterminer la période moyenne d'étalonnage :

- à calculer des périodes de rotation successives ($ST_7$(i) ; $ST_8$(i)) en procédant, pour chacune d'entre-elles, à la sommation de N temps élémentaires ($T_7$(i) ; $T_8$(i)) consécutifs,
- et à traiter les périodes de rotation ($ST_7$(i) ; $ST_8$ (i)) pour déterminer la période moyenne d'étalonnage.

**4.** Procédé d'étalonnage selon la revendication 3, caractérisé en ce que les périodes de rotation successives ($ST_7$(i) ; $ST_8$(i)) sont choisies pour se chevaucher sur au moins un temps élémentaire ($T_7$(i) ; $T_8$ (i)).

**5.** Procédé d'étalonnage selon la revendication 4, caractérisé en que ce que les périodes de rotation successives ($ST_7$(i) ; $ST_8$(i)) sont choisies pour se chevaucher sur (N-1) temps élémentaires ($T_7$(i) ; $T_8$ (i)).

**6.** Procédé d'étalonnage selon une des revendications 3 à 5, caractérisé en ce que le traitement des périodes de rotation ($ST_7$(i) ; $ST_8$(i)) consiste à effectuer le rapport de la somme des périodes de rotation sur le nombre de périodes (j ; k) pour déterminer la période moyenne d'étalonnage ($Tm_7$ ; $Tm_8$).

**7.** Procédé d'étalonnage selon une des revendications 3 à 5, caractérisé en ce que le traitement des périodes de rotation ($ST_7$(i); $ST_8$(i)) consiste à déterminer la période moyenne d'étalonnage (Tm) de manière que le maximum de valeurs de périodes de rotation ($ST_7$(i) ; $ST_8$(i)) se situe dans un intervalle (I) centré autour de cette période moyenne (Tm) et à valider l'étalonnage si le nombre de valeurs de périodes de rotation ($ST_7$(i) ; $ST_8$(i)) contenues dans l'intervalle (I) est supérieur ou égal à un pourcentage prédéterminé du nombre total de valeurs de périodes de rotation ($ST_7$(i) ; $ST_8$(i)).

**8.** Procédé d'étalonnage selon la revendication 7, caractérisé en ce que l'intervalle (I) est compris entre la valeur de la période moyenne (Tm) plus le double d'un écart type standard (ECT) et la valeur de la période moyenne (Tm) moins le double d'un écart type standard (ECT).

**9.** Procédé d'étalonnage selon une des revendications 1 à 8, caractérisé en ce que le pas de mesure (P) est égal à 1.

**10.** Procédé d'étalonnage selon une des revendications 1 à 8, caractérisé en ce que le pas de mesure (P) est égal à N.

**11.** Procédé d'étalonnage selon une des revendications 1 à 10, caractérisé en ce que le coefficient d'étalonnage correspond au rapport des deux périodes moyennes d'étalonnage ($Tm_7$ ; $Tm_8$).

**12.** Procédé d'étalonnage selon une des revendications 1 à 11, caractérisé en ce que, pendant la phase d'étalonnage, le débit du fluide circulant dans la portion de circuit où les débitmètres (7, 8) sont disposés en série est choisi sensiblement identique à un débit nominal de fluide.

**13.** Procédé d'étalonnage selon une des revendications 1 à 12, caractérisé en ce qu'il consiste, pendant l'utilisation des débitmètres (7, 8) à procéder à des phases d'étalonnage espacées dans le temps, pour déterminer des coefficients d'étalonnage successifs.

**14.** Procédé d'étalonnage selon la revendication 13, caractérisé en ce qu'il consiste à :

- définir un coefficient d'étalonnage vrai déterminé par la demi-somme de deux coefficients définis selon deux phases successives d'étalonnage,
- et corriger le volume relatif de liquide mesuré entre les deux phases successives d'étalonnage, par un coefficient de correction correspondant à l'écart entre le coefficient vrai et le premier des deux coefficients considérés.

**15.** Dispositif de contrôle et de traitement pour la mise

en oeuvre du procédé d'étalonnage selon une des revendications 1 à 14, pour des débitmètres équipant un circuit de dialyse (1) d'un rein artificiel, caractérisé en ce qu'il comporte :

- un microprocesseur connecté par une liaison (9) à deux débitmètres (7 ; 8),
- un compteur (11a ; 11b) pour mesurer chaque temps élémentaire ($T_7$(i); $T_8$(i)) séparant deux impulsions émises par chacun des débitmètres,
- un moyen de comptage des impulsions émises pendant les temps élémentaires ($T_7$(i); $T_8$(i)) considérés,
- et des moyens de calcul pour calculer, à partir des temps élémentaires ($T_7$(i) ; $T_8$(i)) mesurés, une période moyenne d'étalonnage ($Tm_7$ ; $Tm_8$) pour chaque débitmètre, et pour calculer, à partir d'une comparaison des périodes moyennes d'étalonnage ($Tm_7$ ; $Tm_8$) calculées, un coefficient d'étalonnage.

**Patentansprüche**

1. Verfahren zur Kalibrierung eines ersten Durchflußmessers (7) mit Hilfe eines zweiten Durchflußmessers (8), das darin besteht, den ersten Durchflußmesser (7) mit dem zweiten Durchflußmesser (8) in Reihe zu schalten, wobei jeder Durchflußmesser ein elektrisches Impulssignal (S) liefert, dessen Frequenz einem gegebenen Wert des Durchflusses eines in einem Kreislauf zirkulierenden Mediums entspricht,
dadurch gekennzeichnet, daß es die Schritte umfaßt:

   - für jeden Durchflußmesser (7, 8) während einer Datenerfassungsphase von einer mittleren Dauer (Ta) jede elementare Zeit (T7(i); T8(i)) zu messen, die zwei um einen bestimmten Meßschritt (P) verschobene Impulse trennt,
   - für jeden Durchflußmesser (7, 8) die Impulse (n; m) zu zählen, die während der Erfassungsphase (Ta) ausgesandt werden;
   - ausgehend von den gemessenen elementaren Zeiten und der Anzahl der gezählten Impulse die mittlere Kalibrierungsperiode (Tm7; Tm8) jedes Durchflußmessers (7, 8) zu berechnen; und
   - ausgehend von einem Vergleich zwischen der mittleren Kalibrierungsperiode (Tm7) des ersten Durchflußmessers (7) und der mittleren Kalibrierungsperiode (Tm8) des zweiten Durchflußmessers (8), die als Bezugswert verwendet wird, einen Kalibrierungsfaktor zu bestimmen.

2. Kalibrierungsverfahren nach Anspruch 1, dadurch gekennzeichnet, daß es darin besteht, die mittlere Kalibrierungsperiode (Tm7; Tm8) zu berechnen, indem das Verhältnis der Summe der elementaren Zeiten (T7(i); T8(i)) zur Anzahl der gezählten Impulse (n; m) gebildet wird.

3. Kalibrierungsverfahren nach Anspruch 1, das an ein Rotameter mit N erzeugten Impulsen pro Rotorumdrehung angepaßt ist, dadurch gekennzeichnet, daß es darin besteht, zur Bestimmung der mittleren Kalibrierungsperiode:

   - aufeinanderfolgende Rotationsperioden (ST7 (i); ST8(i)) zu berechnen, indem für jede von ihnen die Summierung von N aufeinanderfolgenden elementaren Zeiten (T7(i); T8(i)) vorgenommen wird,
   - und die Rotationsperioden (ST7(i); ST8(i)) zu verarbeiten, um die mittlere Kalibrierungsperiode zu bestimmen.

4. Kalibrierungsverfahren nach Anspruch 3, dadurch gekennzeichnet, daß die aufeinanderfolgenden Rotationsperioden (ST7(i); ST8(i)) so gewählt werden, daß sie sich auf wenigstens einer elementaren Zeit (T7(i); T8(i)) überschneiden.

5. Kalibrierungsverfahren nach Anspruch 4, dadurch gekennzeichnet, daß die aufeinanderfolgenden Rotationsperioden (ST7(i); ST8(i)) so gewählt werden, daß sie sich auf (N-1) elementaren Zeiten (T7 (i); T8(i)) überschneiden.

6. Kalibrierungsverfahren nach einem der Ansprüche 3 bis 5, dadurch gekennzeichnet, daß die Verarbeitung der Rotationsperioden (ST7(i); ST8(i)) darin besteht, das Verhältnis der Summe der Rotationsperioden zur Anzahl der Perioden (j; k) zu bilden, um die mittlere Kalibrierungsperiode (Tm7, Tm8) zu bestimmen.

7. Kalibrierungsverfahren nach einem der Ansprüche 3 bis 5, dadurch gekennzeichnet, daß die Verarbeitung der Rotationsperioden (ST7(i); ST8(i)) darin besteht, die mittlere Kalibrierungsperiode (Tm) so zu bestimmen, daß das Maximum der Werte der Rotationsperioden (ST7(i); ST8(i)) in einem Intervall (I) liegt, das um diese mittlere Periode (Tm) zentriert ist, und die Kalibrierung gültig zu machen, wenn die Anzahl der Werte der Rotationsperioden (ST7(i); ST8(i)), die im Intervall (I) enthalten sind, größer oder gleich einem vorgegebenen prozentualen Anteil an der Gesamtzahl der Werte der Rotationsperioden (ST7(i); ST8(i)) ist.

8. Kalibrierungsverfahren nach Anspruch 7, dadurch gekennzeichnet, daß das Intervall (I) zwischen dem

Wert der mittleren Periode (Tm) plus dem Doppelten einer Standardabweichung (ECT) und dem Wert der mittleren Periode (Tm) minus dem Doppelten einer Standardabweichung (ECT) liegt.

**9.** Kalibrierungsverfahren nach einem der Ansprüche 1 bis 8, dadurch gekennzeichnet, daß der Meßschritt (P) gleich 1 ist.

**10.** Kalibrierungsverfahren nach einem der Ansprüche 1 bis 8, dadurch gekennzeichnet, daß der Meßschritt (P) gleich N ist.

**11.** Kalibrierungsverfahren nach einem der Ansprüche 1 bis 10, dadurch gekennzeichnet, daß der Kalibrierungsfaktor dem Verhältnis der zwei mittleren Kalibrierungsperioden (Tm7; Tm8) entspricht.

**12.** Kalibrierungsverfahren nach einem der Ansprüche 1 bis 11, dadurch gekennzeichnet, daß während der Kalibrierungsphase der Durchfluß des Mediums, das in dem Abschnitt des Kreislaufs zirkuliert, in dem die Durchflußmesser (7, 8) in Reihe angeordnet sind, im wesentlichen gleich einem Nenn-Durchfluß des Mediums gewählt wird.

**13.** Kalibrierungsverfahren nach einem der Ansprüche 1 bis 12, dadurch gekennzeichnet, daß es darin besteht, während der Verwendung der Durchflußmesser (7, 8) zeitlich auseinanderliegende Kalibrierungsphasen durchzuführen, um aufeinanderfolgende Kalibrierungsfaktoren zu bestimmen.

**14.** Kalibrierungsverfahren nach Anspruch 13, dadurch gekennzeichnet, daß es darin besteht:

- einen wahren Kalibrierungsfaktor zu definieren, der durch das arithmetische Mittel von zwei in zwei aufeinanderfolgenden Kalibrierungsphasen definierten Faktoren bestimmt wird,
- und das zwischen den zwei aufeinanderfolgenden Kalibrierungsphasen gemessene relative Flüssigkeitsvolumen mittels eines Korrekturfaktors zu korrigieren, welcher der Abweichung zwischen dem wahren Faktor und dem ersten der zwei betrachteten Faktoren entspricht.

**15.** Steuerungs- und Verarbeitungsvorrichtung für die Anwendung des Kalibrierungsverfahrens nach einem der Ansprüche 1 bis 14 für Durchflußmesser, mit denen ein Dialysekreislauf (1) einer künstlichen Niere ausgestattet ist, dadurch gekennzeichnet, daß sie umfaßt:

- einen Mikroprozessor, der über eine Verbindungsleitung (9) mit zwei Durchflußmessern (7; 8) verbunden ist,
- einen Zähler (11a; 11b) zum Messen jeder elementaren Zeit (T7(i); T8(i)), die zwei von jedem der Durchflußmesser ausgesandte Impulse trennt,
- ein Mittel zum Zählen der während der betrachteten elementaren Zeiten (T7(i); T8(i)) ausgesandten Impulse,
- und Berechnungsmittel, um ausgehend von den gemessenen elementaren Zeiten (T7(i); T8 (i)) für jeden Durchflußmesser eine mittlere Kalibrierungsperiode (Tm7; Tm8) zu berechnen, und um ausgehend von einem Vergleich der berechneten mittleren Kalibrierungsperioden (Tm7; Tm8) einen Kalibrierungsfaktor zu berechnen.

## Claims

**1.** A calibration method for a first flowmeter (7) by means of a second flowmeter (8), which consists in connecting in series the first flowmeter (7) to the second flowmeter (8), each flowmeter delivering an electric pulse signal (S) whose frequency corresponds to a given value of flowrate of a fluid circulating in a circuit, the method characterized in that it comprises the steps of :

- measuring for each flowmeter (7, 8), during a data acquisition stage having an average duration (Ta), each elementary period (T7(i); T8(i)) separating two pulses staggered by a specified measurement span;
- counting, for each flowmeter (7, 8), the number of pulses (n, m) emitted during the acquisition stage (Ta),
- calculating the mean calibration period (Tm7; Tm8) for each flowmeter (7, 8), from the measured elementary periods and from the number of counted pulses, and
- determining a calibration coefficient by comparing the mean calibration period (Tm7) of the first flowmeter (7) with the mean calibration period (Tm8) of the second flowmeter (8), this latter period being chosen as reference value.

**2.** The calibration method according to claim 1, characterized in that it consists in calculating the mean calibration period (Tm7; Tm8) by establishing the ratio of the sum of the elementary periods (T7(i)); T8(i)) to the number of counted pulses.

**3.** The calibration method according to claim 1, suitable for a rotary flowmeter with N pulses generated per rotor turn, characterized in that it consists, in order to determine the mean calibration period, in:

- calculating successive rotation periods (ST7(i); ST8(i)), by proceeding for each one of them to

the summation of N consecutive elementary periods (T7(i); T8(i)); and in

- processing the rotation periods (ST7(i); ST8(i)) for determining the mean calculation period.

**4.** The calibration method according to claim 3, characterized in that the successive rotation periods (ST7(i); ST8(i)) are chosen to overlap over at least one elementary period (T7(i); T8(i)).

**5.** The calibration method according to claim 4, characterized in that the successive rotation periods (ST7(i); ST8(i)) are chosen to overlap over (N -1) elementary periods (T7(i); T8(i)).

**6.** The calibration method according to one of claims 3 to 5, characterized in that the processing of the rotation periods (ST7(i); ST8(i)) consists in establishing the ratio of the sum of the rotation periods to the number of periods (j; k), in order to determine the mean calibration period (Tm7; Tm8).

**7.** The calibration method according to one of claims 3 to 5, characterized in that the processing of the rotation periods consists in :

- determining the mean calibration period (Tm), so that the maximum of the values of the rotation periods (ST7(i); ST8(i)) is situated in an interval (I) centered around this mean period (Tm); and
- validating the calibration if the number of measured rotation periods (ST7(i); ST8(i)) contained in the interval (I) is higher than or equal to a predetermined percentage of the total number of the values of the rotation periods (ST7(i); ST8(i)).

**8.** The calibration method according to claim 7, characterized in that the interval (I) lies between the value of the mean period (Tm) plus twice a standard deviation (ECT) and the value of the mean period (Tm) less twice a standard deviation (ECT).

**9.** The calibration method according to one of claims 1 to 8, characterized in that the measurement span (P) is equal to one .

**10.** The calibration method according one of claims 1 to 8, characterized in that the measurement span (P) is equal to N.

**11.** The calibration method according to one of claims 1 to 10, characterized in that the calibration coefficient corresponds to the ratio of the two mean calibration periods (Tm7; Tm8).

**12.** The calibration method according to one of claims 1 to 11, characterized in that, during the calibration stage, the flow rate of the fluid circulating in the portion of circuit where the flowmeters (7, 8) are arranged in series, is chosen substantially identical to a nominal flowrate of the fluid.

**13.** The calibration method according to one of claims 1 to 12, characterized in that it consists, during the use of the flowmeters (7, 8), in proceeding to calibration stages staggered in time, in order to determine successive calibration coefficients.

**14.** The calibration method according to claim 13, characterized in that it consists in :

- defining a true calibration coefficient determined by the halfsum of two coefficients defined according to two successive calibration stages ; and in
- correcting the relative volume of liquid measured between the two successive calibration stages by a correction coefficient corresponding to a deviation between the true coefficient and the first coefficient of the two coefficients considered.

**15.** A control and processing unit for carrying out the calibration method according to one of claims 1 to 14, for flowmeters fitted in the dialysis circuit (1) of an artificial kidney, characterized in that it comprises :

- a microprocessor connected to two flowmeters (7, 8) via a link (9) ;
- a counter (11a; 11b) for measuring each elementary period (T7(i); T8(i)) separating two pulses emitted by each of the flowmeters ;
- means for counting the pulses emitted during the elementary periods (T7(i)); (T8(i)) considered; and
- means for calculating, from the elementary periods (T7(i); T8(i)) measured, a mean calibration period (Tm7; Tm8) for each flowmeter, and for calculating a calibration coefficient from a comparison between the mean calibration periods (Tm7; Tm8).

Fig-1

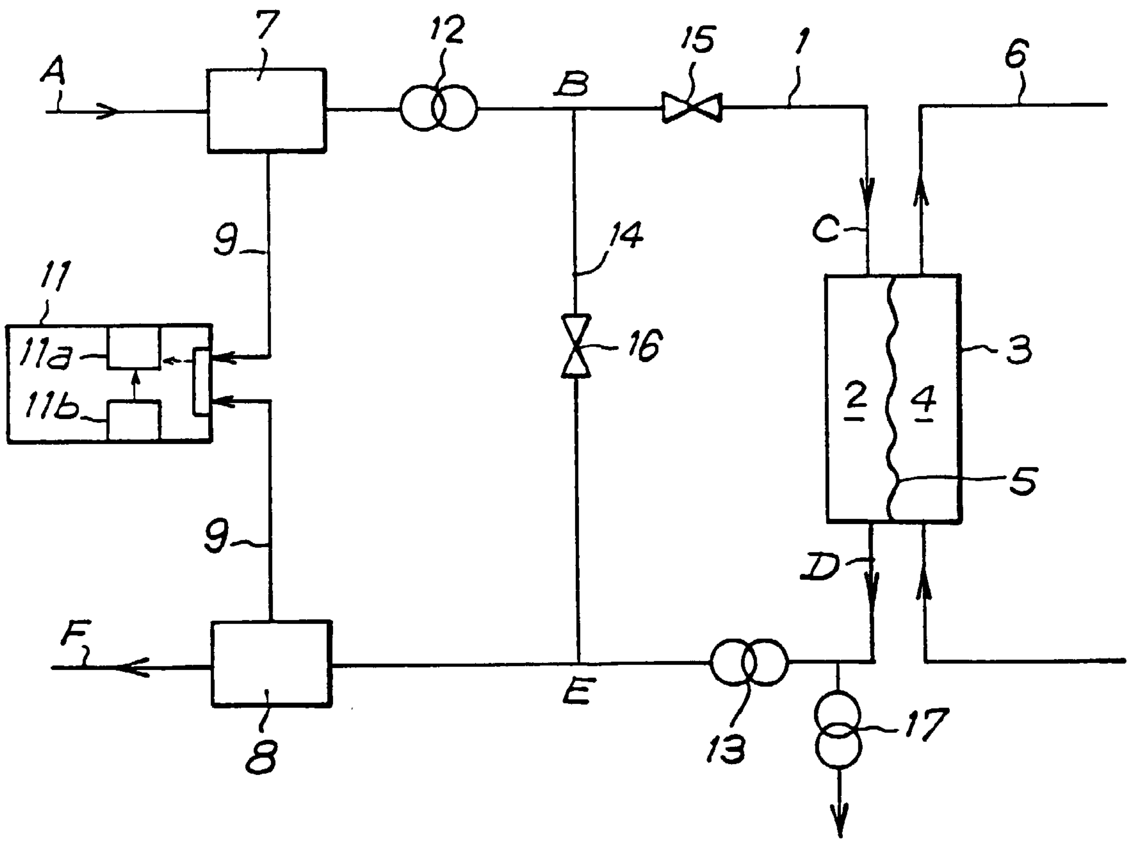

Fig - 2

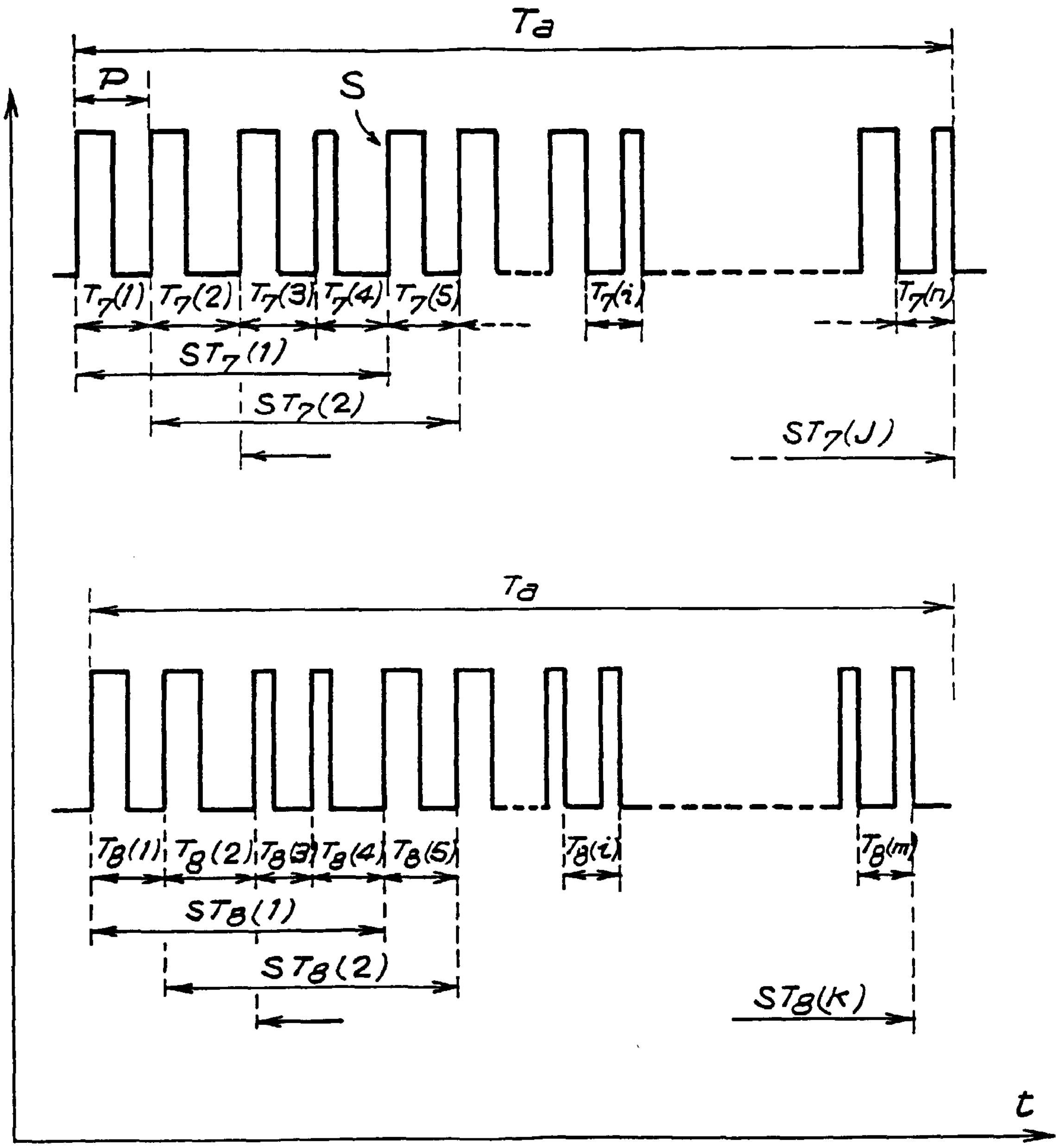
Ta
P
S
T7(1) T7(2) T7(3) T7(4) T7(5) T7(i) T7(n)
ST7(1)
ST7(2)
ST7(J)
Ta
T8(1) T8(2) T8(3) T8(4) T8(5) T8(i) T8(m)
ST8(1)
ST8(2)
ST8(K)
t

Fig-4

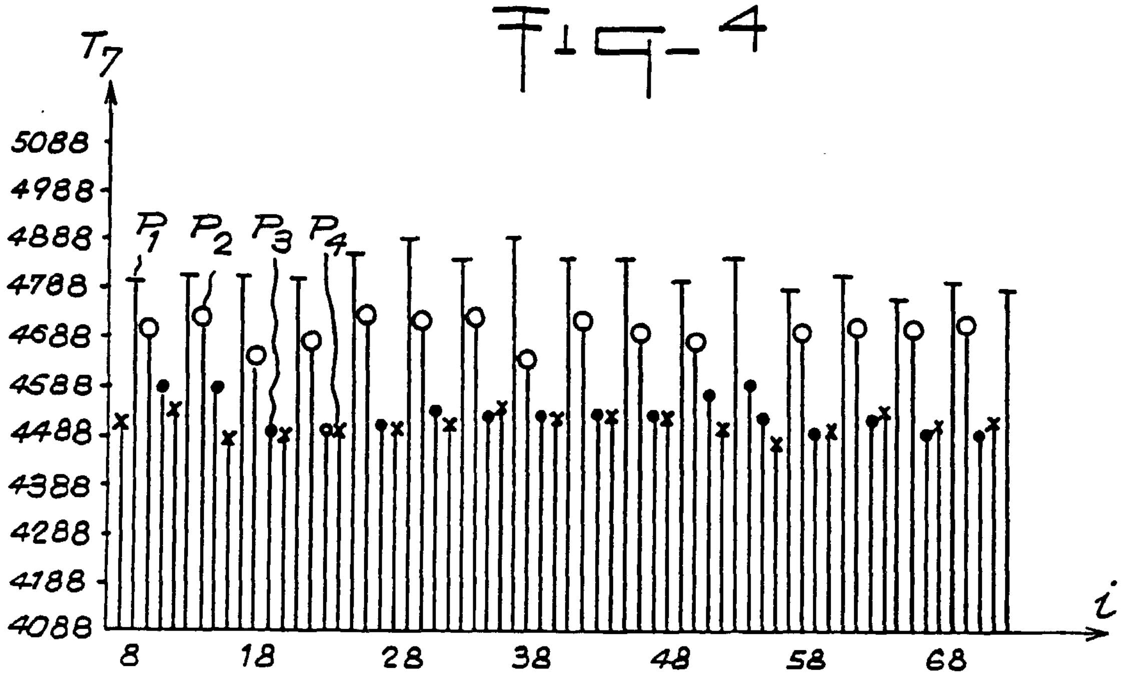
$T_7$
5088
4988
4888
4788
4688
4588
4488
4388
4288
4188
4088
$P_1$ $P_2$ $P_3$ $P_4$
8
18
28
38
48
58
68
i

Fig-3

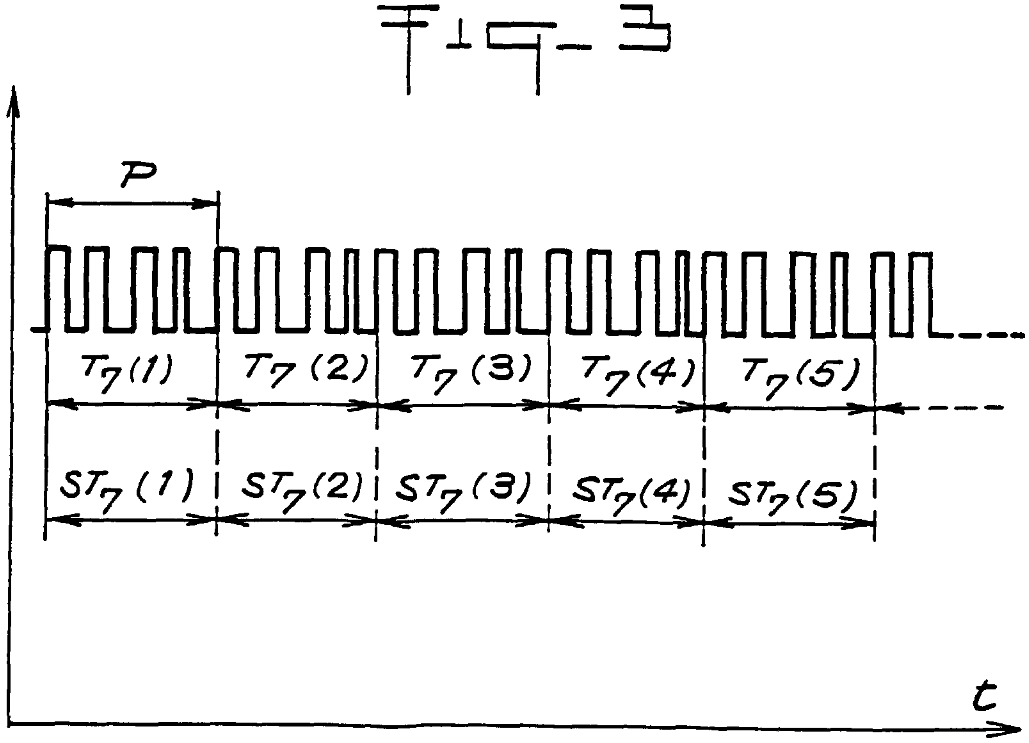
P
$T_7(1)$ $T_7(2)$ $T_7(3)$ $T_7(4)$ $T_7(5)$
$ST_7(1)$ $ST_7(2)$ $ST_7(3)$ $ST_7(4)$ $ST_7(5)$
t

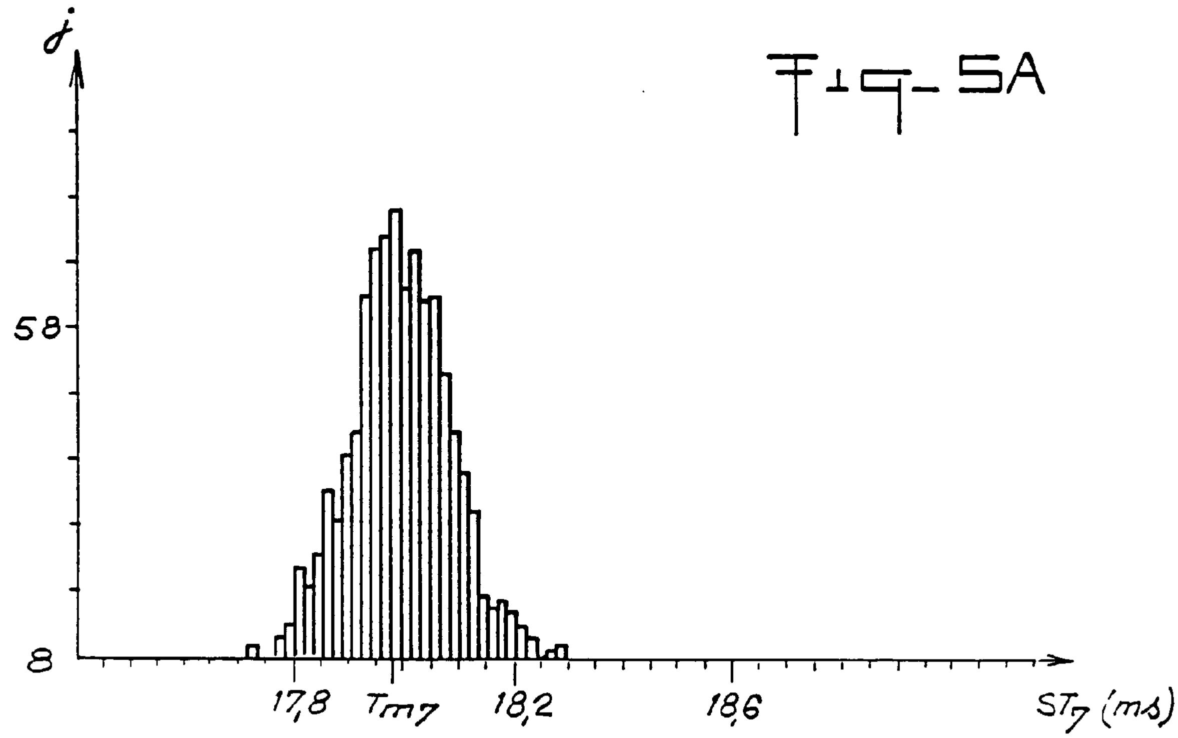
j
58
8
17,8
Tm7
18,2
18,6
ST7 (ms)

Fig. 5A

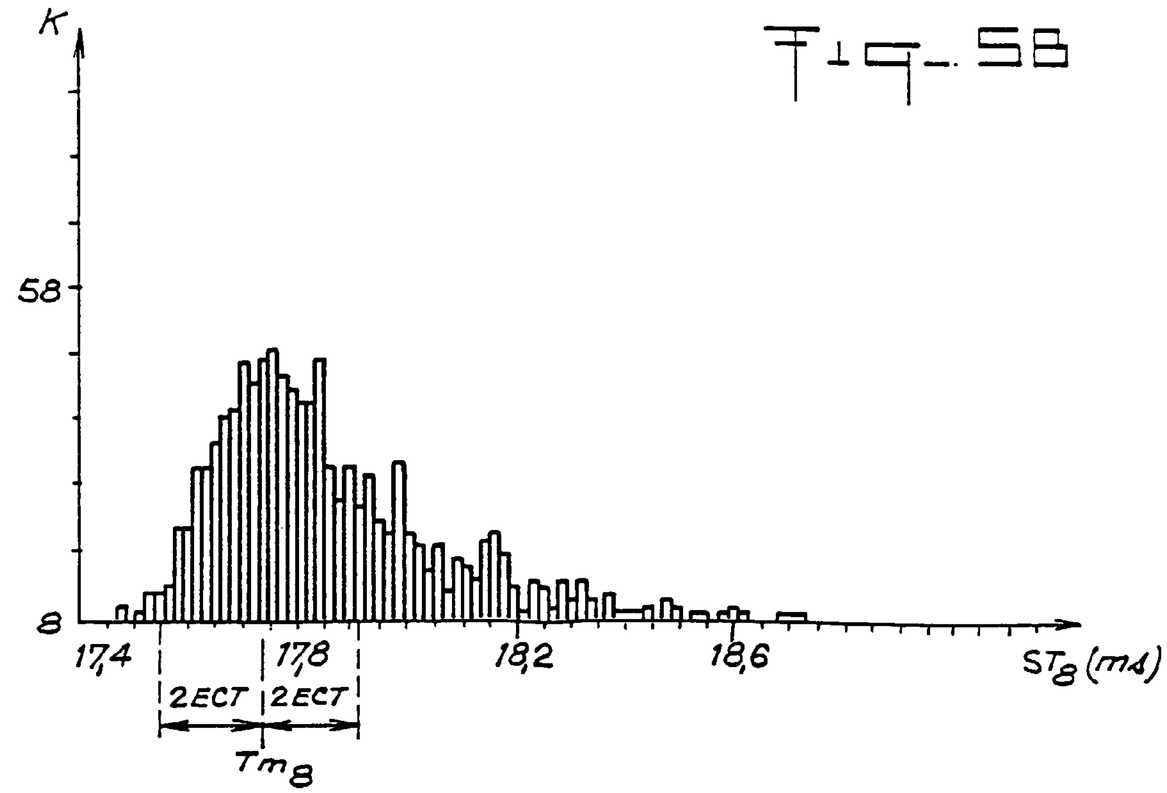
K
58
8
17,4
17,8
18,2
18,6
ST8 (ms)
2ECT
2ECT
Tm8

Fig. 5B

Fig-6

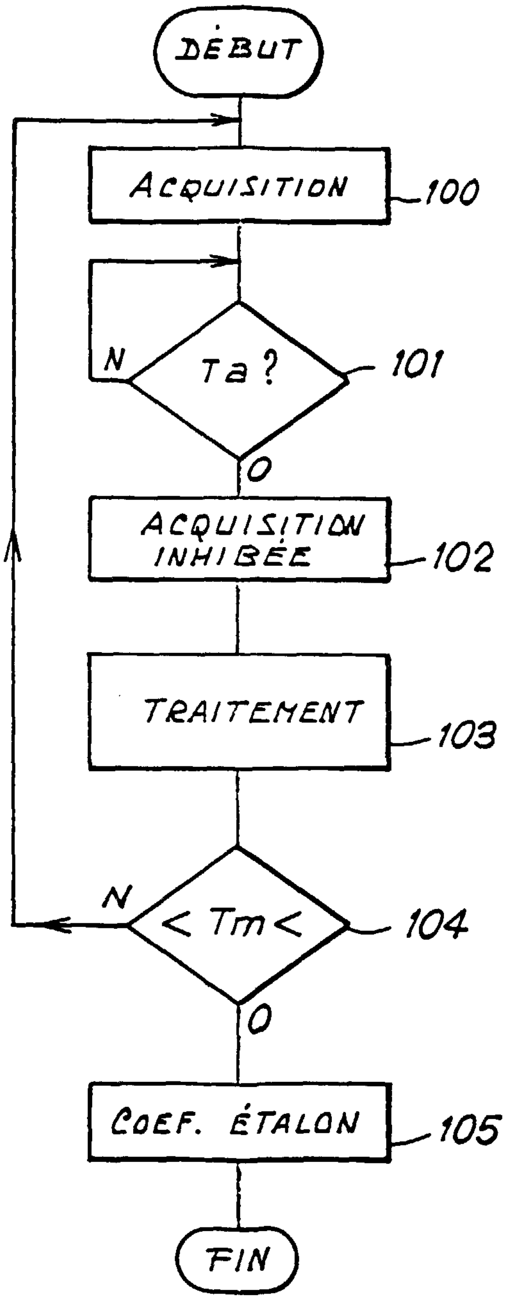
DÉBUT
ACQUISITION
100
Ta ?
N
O
101
ACQUISITION INHIBÉE
102
TRAITEMENT
103
< Tm <
N
O
104
COEF. ÉTALON
105
FIN